## Europäisches Patentamt
## European Patent Office
## Office européen des brevets

(19)

(11) Veröffentlichungsnummer: **0 377 759**

**A1**

(12) ## EUROPÄISCHE PATENTANMELDUNG
### veröffentlicht nach Art. 158 Abs. 3 EPÜ

(21) Anmeldenummer: **89908288.7**

(22) Anmeldetag: **17.05.89**

(86) Internationale Anmeldenummer: **PCT/SU89/00122**

(87) Internationale Veröffentlichungsnummer: **WO 89/12387 (28.12.89 89/30)**

(51) Int. Cl.5: **A01H 4/00, C12N 5/00**

(30) Priorität: **16.06.88 SU 4441705**

(43) Veröffentlichungstag der Anmeldung: **18.07.90 Patentblatt 90/29**

(84) Benannte Vertragsstaaten: **BE CH DE GB LI NL SE**

(71) Anmelder: **ESTONIAN RESEARCH INSTITUTE OF AGRICULTURE AND LAND IMPROVEMENT ul. Teaduse, 1 pos. Saku kharjusky raion Estonskaya SSR, 203400(SU)**

(72) Erfinder: **ROZENBERG, Viive Rudolf ul. Silla, 3 pos. Saku Kharjusky raion Estonskaya SSR, 203400(SU)**
Erfinder: **KOTKAS, Katrin Otto ul. Teaduse, 12-14 pos. Saku Kharjusky raion Estonskaya SSR, 203400(SU)**
Erfinder: **KIISK, Yaan Karl-Lembit ul. Orguse, 4-1 pos. Simuna Rakveresky raion Estonskaya SSR, 202311(SU)**
Erfinder: **SARNET, Vyaino Juhannes ul. Katse-yaama tee, 2-1 pos. Simuna Rakveresky raion Estonskaya SSR, 202311(SU)**

(74) Vertreter: **Nix, Frank Arnold, Dr. Kröckelbergstrasse 15 D-6200 Wiesbaden(DE)**

(54) ## VERFAHREN ZUR VERMEHRUNG UND ZUM PFLANZEN VON KARTOFFELN.

(57) Das vorliegende Verfahren besteht darin, daß man nach der Herstellung von gesunden Sprößlingen in einem Kulturgewebe und ihrer Stecklingspflanzung die Einwurzelung der Mikrostecklinge unter nicht sterilen Bedingungen auf einem Nährboden-Substrat vornimmt, als welches man Niedermoortorf verwendet, in den man Ammophos, Ammoniumnitrat, Kaliumsulfat, Magnesiumsulfat, Eisensulfat, Kupfersulfat, Zinksulfat, Borsäure, Ammoniummolybdat und Schieferasche in entsprechenden Verhältnissen je 1m³ Torf einbringt.

# VERFAHREN ZUR VERMEHRUNG UND AUSPFLANZUNG DER KARTOFFEL

## Gebiet der Technik

Die Erfindung bezieht sich auf die Landwirtschaft, insbesondere auf ein Verfahren zur Vermehrung und Auspflanzung von Kartoffeln, nämlich auf primäre Samenzucht. Sie kann bei der Selektion eines gesunden Ausgangskartoffel-saatgutes sowie bei Selektion neuer Sorten erfolgreich eingesetzt werden.

## Vorhergehender Stand der Technik

Gegenwärtig werden für die Vermehrung von Kartoffel-Jungpflanzgut solche Methoden wie Einwurzelung von Stengelspitzen und blattwinkelständigen Trieben, Vermehrung mit Einlegern, Keimlingen und Keimlingsschnittlingen angewendet.

Die Methode der Einwurzelung von Stengelspitzen und blattwinkelständigen Trieben besteht in folgendem: man schneidet von den virusfreien Mutterpflanzen mit einer Höhe von 20 bis 25 cm die Spitzen mit drei bis vier echten Blättern ab, läßt sie in einer Heteroauxin-Lösung stehen und pflanzt man in den feuchten Boden aus, dann werden die blattwinkelständigen Triebe, die für Auspflanzung verwendet werden, abgetrennt. Der Vermehrungskoeffizient dieser Methode beträgt 1:700.

Die Auspflanzung von Pflanzen erfolgt in Töpfe oder Ziergefäße. Bei der Vermehrung mit Einlegern werden die virusfreien Knollen in Kisten mit Torfboden ausgebreitet und mit einer Torfschicht mit einer Stärke von 2 bis 3 cm oberhalb der Knollenoberfläche zugeschüttet. Nach dem Erscheinen von Sprößlingen mit einer Höhe von etwa 10 cm werden diese von den Knollen abgetrennt, man läßt sie in Töpfen heranwachsen und pflanzt sie in den Boden aus; die Knollen werden mit Torf zwecks wiederholter Bildung von Keimsprossen zugeschüttet. Dies ergibt bis zu fünf Abnahmen. Der Vermehrungskoeffizient bei diesem Verfahren beträgt 1:60--1:90.

Bei Stecklingspflanzung werden die virusfreien Knollen nach der Ernte zum längeren Vorkeimen bei Raumtemperatur und bei mäßiger natürlicher Beleuchtung eingelegt.

20 Tage vor der Auspflanzung in den Boden werden die Keimsprosse von den Knollen abgetrennt, in Schnittlinge mit einer Sproßknolle geschnitten und auf . angefeuchtetem Filterpapier auf einem Blech in einer Schicht ausgebreitet. Nach der Bildung von Wurzeln und Stengelansätzen an jedem Schnittling werden sie auf der Oberfläche angefeuchteten Torfes in Kisten ausgepflanzt und von oben mit Torf zu-geschüttet. In 12 bis 15 Tagen wachsen aus den Schnittlingen die zum Auspflanzen in Gewächshäusern geeigneten Pflanzen heran. Der Vermehrungskoeffizient dieses Verfahrens beträgt 1:770-1:1130.

Die obenbeschriebenen Verfahren sind jedoch bei der Anwendung uneffektiv, da sie einen relativ niedrigen Vermehrungskoeffizienten aufweisen und eine spezielle Vorbereitung, erstens von Mutterpflanzen und Knollen vor Vermehrung und zweitens das Heranwachsen von Pflanzen in Töpfen oder Kisten vor ihrer Auspflanzung in den Treibhausboden verlangen. .

Bekannt ist ebenfalls ein Verfahren zur Vermehrung von Kartoffel-Jungpflanzgut mit Mikroknollen. Gemäß diesem Verfahren werden die Mikroknollen zuerst in Reagenzgläsern in einem Nährboden (in vitro) aufgezogen, sie werden innerhalb von 2 bis 3 Monaten unter bestimmten Bedingungen (Temperatur, Feuchtigkeit) aufbewahrt und dann werden die Mikroknollen entweder in den Gewächshausboden (oder im Freiland) ausgepflanzt, oder man läßt aus den Mikroknollen Stecklinge (in Kisten) heranwachsen, die man seinerseits unter Bedingungen eines Gewächshauses (oder im Freiland) auspflanzt.

Dieses Verfahren weist jedoch einen relativ niedrigen Vermehrungskoeffizienten auf (1:2--1:10). und eine Es verlangt außerdem sterile Bedingungen spezielle Apparatur für die Durchführung der Vermehrung und Aufbewahrung der Knollen. Die Lebensfähigkeit der Mikroknollen ist auch niedrig und übertrifft nicht 85%.

Bekannt ist auch ein Verfahren zur Vermehrung von Kartoffeln (SU, A, 1319800), das die Züchtung von ge-sunden Trieben in einer Gewebekultur, die Mikrostecklingspflan-

- 3 -

zung, die Einwurzelung der Mikrostecklinge und die Auspflanzung der Regenerationspflanzen unter Feldbedingungen in eine Tiefe von 15 bis 20 cm vorsieht. Dabei läßt man die Pflanzen vor dem Auspflanzen auf einem Feld in offenen Reagenzgläsern innerhalb von 4 bis 7 Tagen (bis zu einer Höhe von 23 bis 25 cm) heranwachsen.

Dieses Verfahren ist jedoch uneffektiv bei der Anwendung, weil man erstens die Vermehrung mit Mikrostecklingen unter aseptischen Bedingungen vornimmt, und zweitens wird die Akklimatisierung von Pflanzen vor ihrer Auspflanzung in den Boden (Heranwachsen innerhalb von 4 bis 7 Tagen in offenen Reagenzgläsern) notwendig, und zum dritten haben die                Pflanzen auf einem Feld mit ebener Oberfläche keine günstigen mikroklimatischen Bedingungen für die Entwicklung.

Bekannt ist ebenfalls ein Verfahren zur Vermehrung von Kartoffelpflanzen mit Mikrostecklingen (SU, A, 1025373), das in folgendem besteht. Die gesunden Pflanzen aus dem Reagenzglas schneidet man in Mikrostecklinge, die man wieder in Reagenzgläsern mit Nährboden zwecks Regenerierung der Pflanzen kultiviert und im gleichen Verfahren vermehrt oder in Töpfe mit Niedermoortorf zwecks Erhaltung von Setzlingen ausgepflanzt. Die letzteren werden in Gewächshausboden ausgepflanzt.

Dieses Verfahren ist jedoch bei der Anwendung uneffektiv, da der gesamte Vermehrungsprozeß unter Laborbedingungen oder unter Gewächshausbedingungen verläuft.

Offenbarung der Erfindung

Die Aufgabe der vorliegenden Erfindung besteht in der Schaffung von Bedingungen für eine beschleunigte Vermehrung von Kartoffeln mit Mikrostecklingen auf einem Torfsubstrat zur Erzielung einer maximalen Lebensfähigkeit der Mikrostecklinge und der unmittelbaren Kultivierung von Regenerationskartoffelpflanzen zwecks der Erreichung einer Ernte und einer kurzfristigen Gewinnung von hochqualitativem Jungpflanzgut.

Die gestellte Aufgabe wird dadurch gelöst, daß man im Verfahren zur Vermehrung und Auspflanzung von Kartoffeln,

- 4 -

das die Herstellung von gesunden Trieben in einer Gewebekultur, die Mikrostecklingspflanzung, die Einwurzelung der Mikrostecklinge und die Auspflanzung der Regenerationspflanzen in den Boden . vorsieht, erfindungsgemäß die Einwurzelung der Mikrostecklinge unter nicht sterilen Bedingungen auf einem Nährsubstrat erfolgt, als solches verwendet man Niederungstorf mit Ammophos, Ammoniumnitrat, Kaliumsulfat, Magnesiumsulfat, Eisensulfat, Kupfersulfat, Zinksulfat, Borsäure, Ammoniummolybdat und Schieferasche bei folgendem Verhältnis in kg je 1 m$^3$ Torf:

| | |
|---|---|
| Ammophos | von 0,3 bis 0,6 |
| Ammoniumnitrat | von 0,1 bis 0,2 |
| Kaliumsulfat | von 0,3 bis 0,6 |
| Magnesiumsulfat | von 0,3 bis 0,5 |
| Eisensulfat | von 0,025 bis 0,050 |
| Kupfersulfat | von 0,025 bis 0,050 |
| Zinksulfat | von 0,05 bis 0,015 |
| Borsäure | von 0,005 bis 0,015 |
| Ammoniummolybdat | von 0,001 bis 0,003 |
| Schieferasche | von 5,0 bis 7,0. |

Zweckmäßigerweise sollen die auf Nährbodensubstrat kultivierten Pflanzen in in Furchen gebildete kleine Pflanzlöcher ausgepflanzt werden, wobei die Pflanzenspitzen an der Oberfläche gelassen werden.

Eine solche Ausführung des Verfahrens ermöglicht es, den Vermehrungsprozeß mit Mikrostecklingen infolge des Wegfalls der Notwendigkeit, erstens sterile Räumlichkeiten und zweitens Apparaturen zur Kontrolle und Steuerung der Bedingungen der Vermehrung einzusetzen, wesentlich zu vereinfachen. Es werden außerdem die mikroklimatischen Bedingungen der Kultivierung von Regnerationspflanzen auf dem Feld verbessert.

Das Wesen der Erfindung besteht in folgendem.

Die Verwendung von Niedermoortorf als Substrat bei der Durchführung der Vermehrung unter nicht sterilen Bedingungen ist darauf zurückzuführen, weil er in seiner Struktur für die Gewährleistung guter Bedingungen für die Einwurzelung und Entwicklung von Mikrostecklingspflanzen besonders geeignet ist. Unter Berücksichtigung dessen

- 5 -

aber, daß der Torf einen pH-Wert in einem Bereich von 2,5 bis 3,5 aufweist, soll er neutralisiert werden, das heißt sein pH-Wert soll auf 5,0 bis 5,5 gebracht werden. Für dieses Ziel eignet sich am besten die Schiefereasche. Die übrigen Mikro- und Makroelemente, die in das Substrat eingeführt werden, gewährleisten Bedingungen für eine normale Einwurzelung und Entwicklung der Mikrostecklingspflanzen.

Dabei soll die Vermehrung der Pflanzen zweckmäßigerweise auf einem Substrat in einer Folienrolle vorgenommen werden, da dort besonders geeignete Licht- und Wärmebedingungen für die Durchführung dieses Prozesses vorhanden sind.

Das von uns beschriebene Verhältnis der in das Substrat einzuführenden Elemente bewirkt eine hohe Einwurzelung, Entwicklung und Lebensfähigkeit der Pflanzen bei verschiedener Struktur und verschiedenem Gehalt an Torf. All das beruht auf dem gegenseitigen Gleichgewicht zwischen den einzelnen Mikro- und Makroelementen untereinander im Substrat, weshalb die Pflanzen die oben beschriebenen Elemente in besonders optimalen Verhältnissen assimilieren.

Die Auspflanzung von Pflanzen in den Boden von Furchen gewährleistet ihnen ein günstiges Mikroklima. Verbessert werden auch die Bedingungen der Behäufelung der Pflanzen (mit Boden vom Furchen-rücken) was die Nährfläche jedes Klons vergrößert und dadurch den Kartoffelernteertrag insgesamt erhöht.

Beste Ausführungsvariante der Erfindung

Zur besseren Erläuterung des Wesens der Erfindung wird nachstehend ein konkretes Beispiel für ihre Realisierung angeführt.

Das Verfahren zur Vermehrung und Auspflanzung wird wie folgt durchgeführt.

Die Pflanzen aus Reagenzgläsern werden in Stücke geschnitten, indem man an jedem Abschnitt (Mikrosteckling) einen Blattansatz läßt. Ein Teil des Stengels unterhalb des Blattes wird mit einer Mischung für die Stimulierung des Wachstums und der Einwurzelung behandelt. Dann werden

- 6 -

die Mikrostecklinge in ein Nährbodensubstrat in eine Folienrolle ausgepflanzt, als Nährbodensubstrat verwendet man den
zerkleinerten Niedermoortorf, der beim Brennen anfällt, und
dessen pH-Wert bis auf 5,0...5,5 gebracht wird.

1 m³ Torf setzt man Düngemittel, Mikro- und Makroelemente in folgenden Mengen zu:

| | |
|---|---|
| Ammophos | von 0,3 bis 0,6 kg |
| Ammoniumnitrat | von 0,1 bis 0,2 kg |
| Kaliumsulfat | von 0,3 bis 9,6 kg |
| Magnesiumsulfat | von 0,3 bis 0,6 kg |
| Eisensulfat | von 25 bis 50 g |
| Kupfersulfat | von 25 bis 50 g |
| Zinksulfat | von 5 bis 15 g |
| Borsäure | von 5 bis 7 g |
| Ammoniummolybdat | von 1 bis 3 g |
| Schieferasche | von 5 bis 7 kg. |

Nach der Auspflanzung der Mikrostecklinge wird der
Torf mit Wasser besprengt und die Rolle wird mit einer Polyäthylenfolie abgedeckt.
                                                    regenerieren
In Abhängigkeit von der Sorte ᐯ sich die Pflanzen
(bis zu 15 cm) innerhalb von 20 bis 25 Tagen.
Bei einer weiteren Vermehrung kann man aus jeder Pflanze 4
bis 5 Stecklinge erhalten, die man wieder in einer Rolle
auspflanzt. Die Pflanzen werden bis zum letzten Blatt der
Mikrostecklingspflanzung ausgesetzt, das man in der ersten
Rolle zur weiteren Entwicklung beläßt. Aus jeder aus einem
Mikrosteckling regenerierten Pflanze lassen sich mindestens
3 Mikrostecklingsernten erhalten. Während der letzten Stufe
der Vermehrung werden die Stecklinge in eine Rolle mit
einem Abstand von 7 bis 8 cm ausgepflanzt. Aus diesen Stecklingen erhält man direkt in den Rollen Jungpflanzen mit
                c
gut entwickelten Wurzeln, die im Freiland auf einem Feld
ausgepflanzt werden können. Hierdurch entfällt die Notwendigkeit der Anwendung von Töpfen oder Kisten für die vorläufige Auspflanzung von Jungpflanzen.

Vor der Auspflanzung der Pflanzen auf einem Feld werden
vorher  Furchen gezogen und in deren Boden kleine Pflanzlöcher ausgeführt. Die Pflanzen werden im Loch am besten etwas geneigt aufgestellt und mit Erde zugeschüttet, wobei die Spitzen an der Oberfläche bleiben.

Durch das Befinden am Boden der jeweiligen Furche genießen die Pflanzen günstige klimatische Bedingungen für ihre weitere Entwicklung.

Je nach dem Heranwachsen der Pflanzen wird die Behäufelung der Kartoffeln mit Boden vorgenommen, der am Furchenrücken vorhanden ist.

In der Tabelle 1 wird die Einwurzelung von Kartoffel-Mikrostecklingen bei verschiedenen Zusammensetzungen des Nährbodensubstrats und in der Tabelle 2 werden die Ergebnisse der Qualitätskennziffern von Knollen der ersten Generation und der Lebensfähigkeitskennziffern angeführt.

Tabelle 1

Einfluß des Substrats auf den Prozentsatz der eingewurzelten Stecklinge

| Substrat | Kartoffelsorte | Anzahl der Mikrostecklinge | Einwurzelung der Stecklinge | |
|---|---|---|---|---|
| | | | Stück | in % |
| 1 | 2 | 3 | 4 | 5 |
| I. Variante: | | | | |
| Schieferasche 7 kg | "Ane" | 420 | 417 | 99,3 |
| Ammophos 0,3 kg | "Iygevakollane" | 420 | 413 | 99,5 |
| Ammoniumnitrat 0,1 kg | | | | |
| Kaliumsulfat 0,3 kg | "Eba" | 420 | 415 | 98,8 |
| Magnesiumsulfat 0,3 kg | "Sulev" | 420 | 410 | 97,6 |
| Eisensulfat 50 g | | | | |
| Kupfersulfat 15 g | | | | |
| Zinksulfat 15 g | | | | |
| Borsäure 7 g | | | | |
| Ammoniummolybdat 3 g | | | | |
| II. Variante: | | | | |
| Schieferasche 5 kg | "Ane" | 420 | 416 | 99,0 |
| Ammophos 0,6 kg | "Iygevakollane" | 420 | 420 | 100 |

Fortsetzung der Tabelle 1

| 1 | 2 | 3 | 4 | 5 |
|---|---|---|---|---|
| Ammoniumnitrat 0,2 kg"Eba" | | 420 | 417 | 99,3 |
| Kaliumsulfat 0,6 kg "Sulev" | | 420 | 408 | 97,1 |
| Magnesiumsulfat 0,5 kg | | | | |
| Eisensulfat 25 g | | | | |
| Kupfersulfat 25 g | | | | |
| Zinksulfat 5 g | | | | |
| Borsäure 5 g | | | | |
| Ammoniummolybdat 1 g | | | | |
| Auf Nährboden | | | | |
| (in vitro) | "Ane" | 150 | 150 | 100 |
| | "Iygeva-kollane" | 120 | 120 | 100 |
| | "Eba" | 326 | 324 | 99,4 |
| | "Sulev" | 274 | 270 | 98,5 |

Aus den in der Tabelle 1 angeführten Angaben geht hervor, daß sich der Prozentsatz der Einwurzelung von Mikrostecklingen unter den nicht sterilen Bedingungen in einem Nährbodensubstrat von der Einwurzelung in Kultivierungsgefäßen mit sterilem Nährboden nicht unterscheidet. Das ermöglicht, den Prozeß der Mikrovermehrung von Jungpflanzmaterial zu beschleunigen und die Kosten für die Mikrovermehrung zu reduzieren.

Aus den Angaben der Tabelle 2 ist zu ersehen, daß in dem vorgeschlagenen Verfahren eine hohe Lebensfähigkeit von Pflanzen von 99,3 bis 100% erreicht wird. Die Anzahl der Knollen der ersten Generation je Pflanze betrug (im Durchschnitt) von 40 bis 50 Stück, das durchnittliche Gewicht der Knollen betrug von 25 bis 290 g.

Die vorgeschlagene Technologie ermöglicht es, in 1 Jahr aus einer Pflanze bis zu 5 Mio Pflanzen zu züchten und aus diesen ihrerseits bis zu 45 Millionen Knollen anzubauen.

Auf der Grundlage des Obendargelegten kann man schlußfolgern, daß das vorgeschlagene Verfahren:

- die Vermehrung vereinfacht, was gestattet, dieses in der praktischen Samenzucht zu verwenden;

EP 0 377 759 A1

- 9 -

Tabelle 2

Qualitätskennziffern der regenerierten Pflanzen
und der Knollen der ersten Generation

| Lfd. Nr. | S o r t e | Reifegrad | Anzahl der ausgepflanz-ten Pflanzen (St.) | Lebensfähigkeit, % |
|---|---|---|---|---|
| 1. | "Premier" | frühreifende | 480 | 99,5 |
| 2. | "Andretta" | mittelreifende | 480 | 99,5 |
| 3. | "Ane" | mittelreifende | 480 | 100 |
| 4. | "Eba" | mittelspät-reifende | 480 | 100 |
| 5. | "Sulev" | mittel-spät-reifende | 480 | 99,3 |
| 6. | "Vigri" | spätreifende | 480 | 100 |

Fortsetzung der Tabelle 2

| lfd. Nr. | Ernteerfassung | | |
|---|---|---|---|
| | Durchschnittsgewicht der Klone, g | Variabilität | |
| | | Anzahl der Knollen je eine Pflanze (Stück) | Gewicht der Knollen, g |
| 1. | 1350 | 26...52 | 80...290 |
| 2. | 1440 | 19...27 | 70...210 |
| 3. | 1430 | 24...65 | 40...160 |
| 4. | 1680 | 22...41 | 35...220 |
| 5. | 1520 | 30...58 | 40...190 |
| 6. | 1485 | 27...67 | 20...120 |

- ermöglicht es, neue Sorten schnell zu vermehren und in die Produktion einzuführen;

- die Kartoffelernteerträge erhöht;

- die Kosten für die Produktion von Kartoffelpflanzgut reduziert;

- den Produktionszyklus von Kartoffelpflanzgut wesentlich kürzt.

Gewerbliche Anwendbarkeit

Dieses Verfahren zur Vermehrung und Auspflanzung von

- 10 -

Kartoffeln kann sowohl für die Herstellung von gesundem Samengut als auch für seine beschleunigte Vermehrung angewendet werden. Es kann außerdem für die Selektion und für die umfassende Einführung neuer Sorten genutzt werden. Es kann Anwendung bei der vegetativen Vermehrung von Pflanzen der Zierkulturen finden. Hiermit kann das beanspruchte Verfahren zur Vermehrung und Auspflanzung von Kartoffeln in allen Zweigen des Pflanzeanbaus Anwendung finden, wo der Bedarf an vegetativer Vermehrung besteht.

- 11 -

PATENTANSPRÜCHE:

1. Verfahren zur Vermehrung und Auspflanzung von Kartoffeln, das die Herstellung gesunder Sprößlinge in einem Kulturgewebe, die Mikrostecklingspflanzung, die Einwurzelung der Mikrostecklinge sowie die Auspflanzung von Regenerat-Pflanzen in den Boden vorsieht, d a d u r c h g e - k e n n z e i c h n e t , daß die Einwurzelung der Mikrostecklinge unter nicht sterilen Bedingungen auf einem Nährbodensubstrat vorgenommen wird, als welches Niedermoortorf verwendet wird, in den man Ammophos, Ammoniumnitrat, Kaliumsulfat, Kupfersulfat, Zinksulfat, Borsäure Ammoniummolybdat und Schieferasche bei folgendem Verhältnis in kg je 1 m$^3$ des Torfes eingebracht werden:

| | |
|---|---|
| Ammophos | von 0,3 bis 0,6 |
| Ammoniumnitrat | von 0,1 bis 0,2 |
| Kaliumsulfat | von 0,3 bis 0,6 |
| Magnesiumsulfat | von 0,3 bis 0,5 |
| Eisensulfat | von 0,025 bis 0,050 |
| Kupfersulfat | von 0,025 bis 0,050 |
| Zinksulfat | von 0,005 bis 0,015 |
| Borsäure | von 0,005 bis 0,007 |
| Ammoniummolybdat | von 0,001 bis 0,003 |
| Schieferasche | von 5,0 bis 7,0. |

2. Verfahren nach Anspruch 1, d a d u r c h g e - k e n n z e i c h n e t, daß die im Nährbodensubstrat herangewachsenen Pflanzen in Pflanzlöcher ausgepflanzt werden, die in Furchen gebildet werden, wobei die Pflanzenspitzen an der Oberfläche belassen werden.

# INTERNATIONAL SEARCH REPORT

International Application No  PCT/SU 89/00122

**I. CLASSIFICATION OF SUBJECT MATTER** (if several classification symbols apply, indicate all) ⁶

According to International Patent Classification (IPC) or to both National Classification and IPC

IPC⁵ – A 01 H 4/00, C 12 N 5/00

**II. FIELDS SEARCHED**

| Minimum Documentation Searched ⁷ | |
|---|---|
| Classification System | Classification Symbols |
| IPC⁴ | A 01 H 1/04, 3/00, C 12 N 5/00 |

Documentation Searched other than Minimum Documentation
to the Extent that such Documents are Included in the Fields Searched ⁸

**III. DOCUMENTS CONSIDERED TO BE RELEVANT⁹**

| Category * : | Citation of Document, ¹¹ with indication, where appropriate, of the relevant passages ¹² | Relevant to Claim No. ¹³ |
|---|---|---|
| A | SU, A1, 1276308 (Nauchno-issledovatelsky institut kartofelnogo khozyaistva), 15 December 1986 (15.12.86) | 1-2 |
| A | CIP CIRCULAR, volume 13, No 4, December 1985, "International Potato Center", (Peru), pages 1-5 | 1-2 |
| A | Annals of Botany, 53, 1984, "Annals of Botany Company"(USA), G. Hussey et al.:"Factors Affecting the Formation of In vitro Tubers of Potato (Solanum tuberosum L.) , pages 565-578 | 1-2 |

---

\* Special categories of cited documents: ¹⁰

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

**IV. CERTIFICATION**

| Date of the Actual Completion of the International Search | Date of Mailing of this International Search Report |
|---|---|
| 15 August 1989 (15.08.89) | 02 October 1989 (02.10.89) |
| International Searching Authority | Signature of Authorized Officer |
| ISA/SU | |

Form PCT/ISA/210 (second sheet) (January 1985)